# EUROPÄISCHE PATENTSCHRIFT

(11) **EP 0 295 527 B1**
(45) Veröffentlichungstag und Bekanntmachung des Hinweises auf die Patenterteilung: **02.03.1994**
(21) Anmeldenummer: 88109016.1
(22) Anmeldetag: 06.06.1988
(51) Int. Cl.: C07C 315/04, C07C 317/04, C07C 317/14

(54) **Verfahren zur Herstellung von (3-Aminophenyl)(2-hydroxyethyl)sulfon**
Process for the preparation of 3-aminophenyl-2-hydroxyethylsulfon
Procédé de préparation de 3-aminophényl-2-hydroxyéthylsulfon

(30) Priorität: 17.06.1987 DE 3720213
(43) Veröffentlichungstag der Anmeldung: 21.12.1988
(73) Patentinhaber: BAYER AG, 51368 Leverkusen (DE)
(72) Erfinder: Herd, Karl-Josef, Dr., D-5068 Odenthal-Holz (DE)

(56) Entgegenhaltungen:
- EP-A- 0 092 909
- EP-A- 0 107 614
- DE-A- 2 240 849
- DE-A- 3 305 654
- PATENT ABSTRACTS OF JAPAN, Band 8, Nr. 7 (C-204)(1444), 12. Januar 1984; & JP-A-58174355
- ADVANCED ORGANIC CHEMISTRY, THIRD EDITION, J. MARCH. PAGE 392
- CA 94: 46889r

## Beschreibung

Gegenstand der vorliegenden Erfindung ist ein Verfahren zur Herstellung des Sulfons der Formel 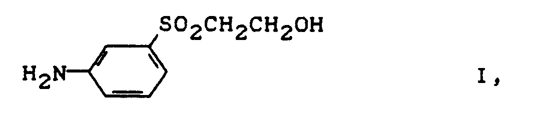
das dadurch gekennzeichnet ist, daß man ein Nitrosulfon der Formel 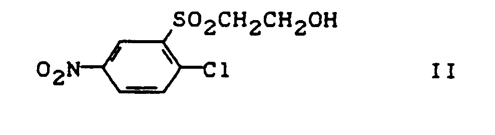
bzw. 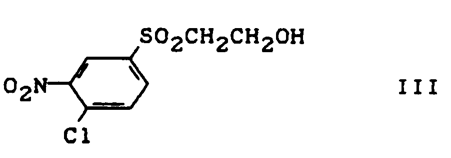
bzw. ein Gemisch aus II und III in Gegenwart von Hydrierungskatalysatoren mit Wasserstoff behandelt.

Die Umsetzung erfolgt vorzugsweise in wäßrigem oder wäßrig-organischem Medium gegebenenfalls in Gegenwart inerter Dispergier- bzw. Emulgiermittel.

Als organische Lösungsmittel kommen dabei insbesondere wassermischbare wie niedermolekulare aliphatische Alkohole und deren Ether, weiterhin Ether wie Dioxan und Tetrahydrofuran in Frage. Sie werden gegebenenfalls in Mengen von etwa 5 bis 25 Gew.-% eingesetzt.

Die Reaktion wird bei Temperaturen von etwa 40 - 120°C, vorzugsweise 60 - 80°C durchgeführt.

Der pH-Wert soll während der Reaktion im Bereich 5 - 8, vorzugsweise im Neutralbereich liegen. Dies läßt sich durch Zusatzt von Puffersubstanzen, insbesondere Phosphaten wie Mischungen aus primären, sekundären und tertiären Alkaliphosphaten, Boraten, Acetaten oder Alkalihydrogencarbonaten oder Alkalicarbonaten erreichen.

Die erforderliche Menge an Puffersubstanzen läßt sich leicht durch Vorversuche ermitteln. Im Falle der Alkalihydrogencarbonate verwendet man zweckmäßigerweise äquimolare Mengen, bezogen auf (II) bzw. (III).

Geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxihydrate handeln. Zusätzlich können die Metalle Kupfer, Vanadin, Molybdän, Chrom und/oder Mangan, sowie Verbindungen dieser Metalle zugegen sein.

Die Hydrierkatalysatoren können ausschließlich oder überwiegend aus wasserstoffübertragenden Substanzen bestehen, diese können aber auch auf Trägermaterialien aufgebracht sein. Als Trägermaterialien für die wasserstoffübertragenden Substanzen kommen beispielsweise in Frage: anorganische Materialien wie Kieselgur, Kieselsäure, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthatische Verbindungen mit hohen Molekulargewichten wie Seide, Polyamide, Polystyrole, Zellstoff oder Polyurethane. Bevorzugt sind anorganische Trägermaterialien. Das Trägermaterial kann beispielsweise in Form von Kugeln, Strängen, Fäden, Zylindern, Polygonen oder in Pulverform vorliegen.

Derartige Trägerkatalysatoren können im allgemeinen 0,5 bis 50 Gew.-%, vorzugsweise 1 bis 10 Gew.-% der wasserstoffübertragenden Substanz, bezogen auf die Gesamtmasse des Trägerkatalysators, enthalten. Die wasserstoffübertragende Substanz kann dabei homogen im Trägermaterial vorteilt sein, bevorzugt sind jedoch Katalysatoren, in deren äußerer Schicht oder auf deren Oberfläche die wasserstoffübertragende Substanz abgelagert ist. Die Herstellung und die Formgebung von Katalysatoren, die im erfindungsgemäßen Verfahren Verwendung finden können, kann in bekannter Weise erfolgen (siehe beispielsweise Houben-Weyl, Methoden der organischen Chemie, Band IV, 1c, Teil I, S. 16-26, Georg Thieme-Verlag, Stuttgart 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid.

Bevorzugte Hydrierkatalysatoren, die ausschließlich oder überwiegend aus wasserstoffübertragender Substanz bestehen, sind beispielsweise oxidische Katalysatoren wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Aklalihydriden, Alkaliboranaten, Metallalkylen, Hydrazin, Formaldehyd, Wasserstoff oder elektropositiveren Metallen herstellbare Schwarzkatalysatoren, wie Palladium/Schwarz, Platin/Schwarz und Rhodium/Schwarz.

Besonders bevorzugte Katalysatoren für das erfindungsgemäße Verfahren sind Palladium auf Kohle, Palladium auf Aluminiumoxid, Palladium auf Kieeselsäure und Palladium auf Calciumcarbonat sowie Raney-Nickel.

Die Katalysatormenge beträgt im allgemeinen etwa 0,1 bis 10 Gew.-%, vorzugsweise 0,1 bis 5 Gew.-%, bezogen auf (II) bzw. (III).

Der Wasserstoffdruck beträgt etwa 1 bis 120 bar, vorzugsweise 5 bis 40 bar.

Die für das erfindungsgemäße Verfahren erforderliche Reaktionszeit ist abhängig von der Reaktionsgeschwindigkeit, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Die katalytische Aktivität der Hydrierkatalysatoren bleibt bei der Durchführung des erfindungsgemäßen Verfahrens im allgemeinen weitgehand erhalten, so daß diese bei diskontinuierlicher Arbeitsweise wiederholt eingesetzt werden kann und bei kontinuierlicher Arbeitsweise längere Zeit in Gebrauch bleiben können.

In einer einfachen diskontinuierlichen Ausführungsform kann das erfindungsgemäße Verfahren beispielsweise wie folgt durchgeführt werden: Ein mit einer Rühr- oder Mischeinrichtung versehener, temperierbarer Autoklav wird mit dem einzusetzenden (II) bzw. (III), dem Hydrierkatalysator und Wasser bzw. Wasser-Lösungsmittel-Gemisch beschickt. Danach wird Wasserstoff bis zum gewünschten Druck aufgedrückt und das Gemisch unter intensiver Durchmischung auf die gewünschte Reaktionstemperatur erhitzt. Der Reaktionsverlauf läßt sich leicht durch Messung des Wasserstoffverbrauchs verfolgen. Während der Reaktion verbrachter Wasserstoff kann kontinuierlich oder diskontinuierlich nachdosiert werden. Die Hydrierung wird abgebrochen, wenn die gewünschte Menge Wasserstoff aufgenommen worden ist. Der Abbruch der Hydrierung kann durch Abkühlung, Beendigung der Durchmischung, Entspannung und/oder Beseitigung der Wasserstoffatmosphäre erfolgen. Die Aufarbeitung des Reaktionsgemisches kann beispielsweise so erfolgen, daß man zunächst den Katalysator abfiltriert und die verbleibende Reaktionslösung aufkonzentriert sowie kristallisierende Produkte durch Absaugen isoliert. Das erfindungsgemäße Verfahren kann auch kontinuierlich durchgeführt werden.

Das Verfahren kann ein- oder zweistufig durchgeführt werden. Beim zweistufigen Verfahren wird dabei zunächst bei etwa 40°C in wäßrigem Medium die Nitrogruppe reduziert, anschließend wird nach Zusatz säurebindender Puffersubstanzen oberhalb 50°C reduktiv dehalogeniert. Bevorzugt wird einstufig gearbeitet.

Gegenstand der vorliegenden Erfindung ist auch ein Verfahren zur Herstellung von (I), dadurch gekennzeichnet, daß man 2-Chlor-5-nitrobenzolsulfochlorid mit Natriumsulfit zur 2-Chlor-5-nitrobenzolsulfinsäure umsetzt, diese anschließend mit Ethylenoxid zu (II) umsetzt und dieses in Gegenwart von Hydrierungskatalysatoren mit Wasserstoff behandelt.

Gegenüber dem vorbekannten Verfahren zur Herstellung von (I), nämlich Umsetzung von 3-Nitrobenzolsulfochlorid zu 3-Nitrobenzolsulfinsäure wie in Houben-Weyl: Methoden oder organischen Chemie, Bd. IX, S. 307 (1955) beschrieben, Reaktion mit Ethylenoxid und anschließende Reduktion der Nitrogruppe gemäß DE-A 2 240 849, weist das erfindungsgemäße Verfahren den überraschenden Vorteil auf, höhere Ausbeuten und weniger schwer abtrennbare Nebenprodukte zu liefern.

(I) ist ein wertvolles Zwischenprodukt für die Herstellung von Farbstoffen. Es wird beispielsweise als Diazokomponente bei der Herstellung von Azofarbstoffen eingesetzt, insbesondere nach vorheriger Sulfatierung zum Sulfatoethylsulfonyl-anilin.

Aus DE-A 3 305 654 ist ein Verfahren zur Herstellung von 3,3'-Diaminodiphenylsulfonen aus den entsprechenden Dinitro-halogen-sulfonen bekannt.

### Beispiel 1

### Natrium-2-chlor-5-nitrobenzolsulfinat

1.000 g (3,8 mol) einer frisch bereiteten ca. 40 %igen wäßrigen Natriumbisulfitlösung und 500 g Eis werden mit ca. 200 ml konz. Natronlauge auf pH 7,5 gestellt. Durch Zugabe von ca. 400 g Eis wird auf 10°C abgekühlt. Portionsweise werden 1,47 kg (3,8 mol) 2-Chlor-5-nitrobenzolsulfonsäurechlorid in Form einer feuchten Paste bei 10 - 15°C eingetragen. Die Temperatur wird durch Zugabe von 1,6 kg Eis und der pH-Wert durch Zugabe von 380 ml Natronlauge bei 7 -7,5 konstant gehalten. Es wird solange nachgerührt, bis sich der pH-Wert praktisch nicht mehr ändert. Der Zeitaufwand für Eintragen und Nachrühren beträgt etwa 4 Stunden. Man stellt auf pH 8,5 und gibt 15 g eines üblichen Filterhilfsmittels zu. Nach einer kurzen Nachrührzeit wird geklärt und mit ca. 300 ml Wasser nachgewaschen. Es wird mit 1,3 kg Kochsalz ausgesalzen, 1 Stunde nachgerührt und abgepreßt. Es werden 1,35 kg feuchtes 2-Chlor-5-nitrobenzolsulfinat isoliert. Die HFC-Analyse liefert einen Gehalt von 58,4 %, was einer Ausbeute von 85 % d. Th. entspricht. Das Produkt enthält ca. 2 % 2-Chlor-5-nitrobenzolsulfonat.

### (2-Chlor-5-nitrophenyl)(2-hydroxyethyl)sulfon

1.045 g (2,5 mol) 2-Chlor-5-nitrobenzolsulfinat in Form einer 58,4 %igen feuchten Paste und 1.250 ml Wasser werden in ein mit Stickstoff gespültes Reaktionsgefäß eingefüllt und der pH-Wert zwischen 7 und 8 eingestellt. Die Lösung wird unter Stickstoff auf 60°C erwärmt. Es werden langsam insgesamt 550 g Ethylenoxid eingeleitet und dabei der pH durch Zudosieren von 25 %iger Schwefelsäure zwischen 6 - 8 gehalten. Es werden insgesamt 1,02 kg Schwefelsäure verbraucht. Die Reaktion ist nach ca. 6 - 7 Stunden beendet. Es wird bei pH 7 und 85°C für 1 Stunde Stickstoff durchgeleitet und dann auf Raumtemperatur abgekühlt. Nach 4 Stunden Rühren bei 20°C wird abgesaugt und der Niederschlag zweimal mit 500 ml Wasser geswaschen. Es werden 775 g feuchte Paste bzw. nach dem Trocknen bei 60 - 70°C i. Vak. 602 g (2-Chlor-5-nitrophenyl)(2-hydroxyethyl)sulfon erhalten. Nach C,H,N-Analyse, Nitrogruppen-, Chlor- und Schwefelbestimmung ist das Produkt 98 %ig, woraus sich eine Ausbeute von 90,5 % d. Th. berechnet.

### (3-Aminophenyl)(2-hydroxyethyl)sulfon

271 g (1 mol) des obigen (2-Chlor-6-nitrophenyl)(2-hydroxyethyl)sulfons werden im 3 1-Autoklaven in 1,8 l Wasser angerührt und mit 85 g festem Natriumhydrogencarbonat und 1 ml eines Emulgators (Polyether auf Basis Laurylalkohol und Ethylenoxid) versetzt. Es wird auf 70°C aufgeheizt. Man fügt 20 g Raney-Nickel, das aus einer 50 : 50-Aluminium-Nickellegierung frisch bereitet wurde, zu und drückt ca. 40 bar Wasserstoff auf. Bei einer Reaktionstemperatur von 70 -75°C wird der Wasserstoffdruck im Bereich zwischen 20 - 25 bar gehalten. Ein zusätzlicher Druckanstieg durch CO₂-Freisetzung ist zu berücksichtigen. Wenn nach ca. 2 - 3 Stunden der Wasserstoffverbrauch deutlich abfällt, wird eine Probe entnommen und chromatographisch (Kieselgel auf Alufolie; Fließmittel: Aceton / Ligroin = 1 : 1) auf Vollständigkeit der Dehalogenierung geprüft. Ist die Reduktion beendet, wird der Autoklav entspannt, mit Stickstoff gespült und die warme Reaktionsmischung über eine Drucknutsche geklärt, wobei der Katalysator als Filterrückstand zurückbleibt.

Das Filtrat wird am Rotationsverdampfer bei 60°C/20 Torr aufkonzentriert. Die resultierenden 263 g eines Natriumchloridhaltigen Öls, das beim Abkühlen auf 0 - 5°C kristallin erstarrt, erweisen sich als praktisch einheitliches (3-Aminophenyl)(2-hydroxyethyl)sulfon. Das Produkt ist nach Gehaltsbestimmung ca. 71 %ig, was einer Ausbeute von 93 % d. Th. entspricht.

### Beispiel 2

34,2 g feuchte Paste (2-Chlor-5-nitrophenyl)(2-hydroxyethyl)sulfon aus Beispiel 1 [bzw. eine äquimolare Menge an (4-Chlor-3-nitrophenyl)(2-hydroxyethyl)sulfon] werden in 250 ml Wasser und 50 ml einer Phosphatpufferlösung, die durch Lösen von 0,5 mol Dinatriumphosphat und 0,33 mol Mononatriumphosphat in 1 l Wasser bereitet wurde, angerührt. Nach Zugabe von 1 - 2 g eines nichtionischen Emulgators und 1,5 g Raney-Nickel, das aus einer 70 : 30-Aluminium-Nickellegierung hergestellt wurde, erwärmt man auf 80°C. Danach werden 20 bar Wasserstoff aufgedrückt. Die Wasserstoffzufuhr wird im folgenden so gesteuert, daß der Druck konstant 10 - 15 bar beträgt. Nimmt der Wasserstoffverbrauch deutlich ab, wird eine Probe entnommen und chromatographisch (s. Beispiel 1) auf Vollständigkeit der Reduktion überprüft. Ist gegebenenfalls noch (5-Amino-2-chlorphenyl)(2-hydroxyethyl)sulfon [bzw. (3-Amino-4-chlorphenyl)(2-hydroxyethyl)sulfon] nachweisbar, wird nochmals ca. 0,5 - 1,0 g Katalysator nachgesetzt und erneut bei 80°C und 20 bar hydriert. Ist die Reduktion und Dehalogenierung beendet, wird der Autoklav entspannt, mit Stickstoff gespült und die warme Reaktionslösung durch Absaugen vom Katalysator abgetrennt. Es wird abgekühlt, mit Salzsäure sauer gestellt und der Gehalt der Lösung an (3-Aminophenyl)(2-hydroxyethyl)sulfon mittels Diazotierung mit 10 %iger Natriumnitritlösung ermittelt. Die Ausbeute beträgt ca. 90 % d. Th.. Die so erhaltene Lösung des (3-Aminophenyl)(2-hydroxyethyl)sulfons kann in dieser Form direkt weiterverarbeitet werden; sie kann z. B. diazotiert werden und die Diazoniumsalzlösung, wie in der japanischen Patentanmeldung 05667/70 beschrieben, auf 7-Acetylamino-1-naphthol-3-sulfonsäure gekuppelt werden.

### Beispiel 3

102,6 g feuchte Paste (2-Chlor-5-nitrophenyl)(2-hydroxyethyl)sulfon aus Beispiel 1 [bzw. eine äquimolare Menge an (4-Chlor-3-nitrophenyl)(2-hydroxyethyl)sulfon] werden in 250 ml Wasser und 50 ml der unter Beispiel 2 beschreibenen Phosphatpufferlösung angerührt. Man fügt eine geringe Menge eines nichtionisichen Emulgators und 5 g Palladium / Kohle-Katalysator zu. Die Hydrierung mit Wasserstoff wird dann bei 70°C und ca. 40 bar im Autoklaven durchgeführt. Die Reduktion wird chromatographisch überprüft. Nach der Hydrierung sowie Entspannung und Spülung des Reaktors wird auf 85°C aufgeheizt und über eine Drucknutsche geklärt. Das Filtrat wird auf 0°C gekühlt und das kristalline (3-Aminophenyl)(2-hydroxyethyl)sulfon durch Filtration isoliert. Man erhält nach dem Trocknen 53 g einheitliches Produkt.

## Patentansprüche

1. Verfahren zur Herstellung des Sulfons der Formel 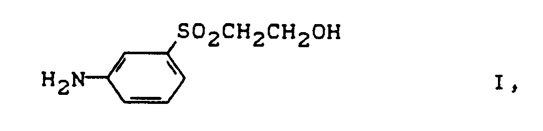 dadurch gekennzeichnet, daß man ein Nitrosulfon der Formel 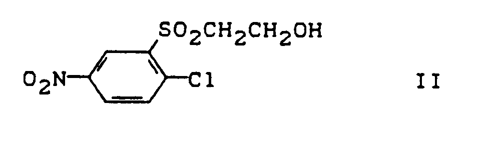 bzw. 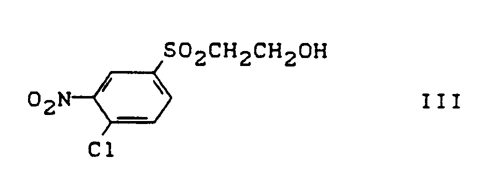 bzw. ein Gemisch aus II und III in Gegenwart von Hydrierungskatalysatoren mit Wasserstoff behandelt.

2. Verfahren gemäß Anspruch 1, dadurch gekennzeichnet, daß die Umsetzung in wäßrigem oder wäßrig-organischem Medium erfolgt.

3. Verfahren gemäß Ansprüchen 1 und 2, dadurch gekennzeichnet, daß die Umsetzung bei Temperaturen von 40 bis 120°C und pH-Werten von 5 bis 8 erfolgt.

4. Verfahren gemäß Ansprüchen 1 bis 3, dadurch gekennzeichnet, daß man als Hydrierungskatalysatoren Nickelkatalysatoren, insbesondere Raney-Nickel oder Pd-Trägerkatalysatoren verwendet.

5. Verfahren zur Herstellung des Sulfons der Formel 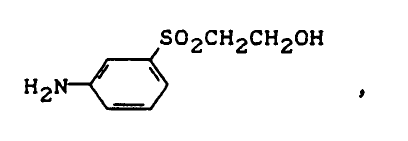 dadurch gekennzeichnet, daß man 2-Chlor-5-nitrobenzolsulfochlorid mit Natriumsulfit zur 2-Chlor-5-nitrobenzolsulfinsäure umsetzt, danach mit Ethylenoxid zu 2-Chlor-5-nitrophenyl-2-hydroxyethyl-sulfon und dieses anschließend in Gegenwart von Hydrierungskatalysatoren mit Wasserstoff behandelt.

## Claims

1. Process for the preparation of the sulphone of the formula 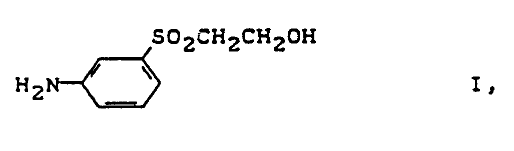 characterized in that a nitrosulphone of the formula 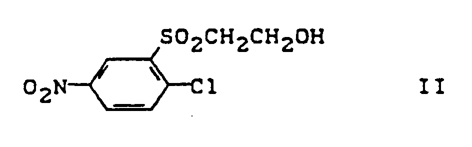 or 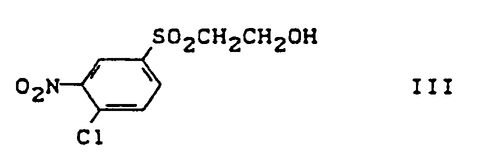 or a mixture of II and III is treated with hydrogen in the presence of hydrogenation catalysts.

2. Process according to Claim 1, characterized in that the reaction is carried out in an aqueous or aqueous organic medium.

3. Process according to Claims 1 and 2, characterized in that the reaction is carried out at temperatures of 40 to 120°C and pH values of 5 to 8.

4. Process according to Claims 1 to 3, characterized in that the hydrogenation catalysts used are nickel catalysts, in particular Raney nickel or Pd supported catalysts.

5. Process for the preparation of the sulphone of the formula 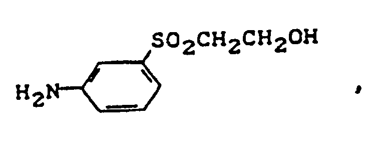 characterized in that 2-chloro-5-nitrobenzenesulphochloride is reacted with sodium sulphite to give 2-chloro-5-nitro-benzenesulphinic acid, the product is then reacted with ethylene oxide to give 2-chloro-5-nitrophenyl 2-hydroxyethyl sulphone and the latter is then treated with hydrogen in the presence of hydrogenation catalysts.

## Revendications

1. Procédé de production de la sulfone de formule 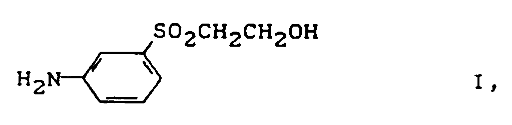 caractérisé en ce qu'on traite avec de l'hydrogène, en présence de catalyseurs d'hydrogénation, une nitrosulfone de formule 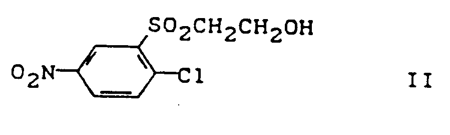 ou 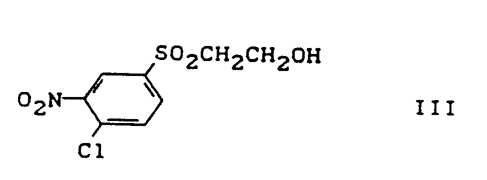 ou un mélange de II et III.

2. Procédé suivant la revendication 1, caractérisé en ce qu'on conduit la réaction en milieu aqueux ou aqueux-organique.

3. Procédé suivant les revendications 1 et 2, caractérisé en ce qu'on conduit la réaction à des températures de 40 à 120°C et à des pH de 5 à 8.

4. Procédé suivant les revendications 1 à 3, caractérisé en ce qu'on utilise comme catalyseurs d'hydrogénation des catalyseurs à base de nickel, notamment du nickel de Raney ou des catalyseurs au palladium fixé sur un support.

5. Procédé de production de la sulfone de formule 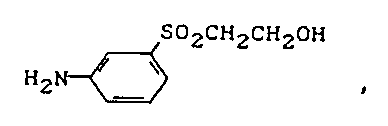 caractérisé en ce qu'on fait réagir le sulfochlorure de 2-chloro-5-nitrobenzène avec du sulfite de sodium pour former de l'acide 2-chloro-5-nitrobenzène-sulfinique, puis avec de l'oxyde d'éthylène pour former la 2-chloro-5-nitrophényl-2-hydroxyéthylsulfone et on traite ensuite cette dernière avec de l'hydrogène en présence de catalyseurs d'hydrogénation.
